# EUROPÄISCHE PATENTSCHRIFT

(11) **EP 3 288 645 B1**
(45) Veröffentlichungstag und Bekanntmachung des Hinweises auf die Patenterteilung: **16.10.2019**
(21) Anmeldenummer: 16721658.9
(22) Anmeldetag: 18.04.2016
(51) Int. Cl.: A61K 8/39, A61K 8/86, A61Q 19/10, A61K 8/92, A61K 8/02, A61K 8/06, A61Q 1/14

(54) **NIEDRIGVISKOSE ÖL-IN-WASSER EMULSIONEN FÜR KOSMETISCHE ANWENDUNGEN**
LOW-VISCOSITY OIL-IN-WATER EMULSIONS FOR COSMETIC APPLICATIONS
ÉMULSIONS HUILE-DANS-L'EAU À FAIBLE VISCOSITÉ POUR APPLICATIONS COSMÉTIQUES

(30) Priorität: 28.04.2015 EP 15165443
(43) Veröffentlichungstag der Anmeldung: 07.03.2018
(73) Patentinhaber: BASF SE, 67056 Ludwigshafen am Rhein (DE)
(72) Erfinder: HLOUCHA, Matthias, 50677 Köln (DE); SEIDLER, Stefanie, 40597 Düsseldorf (DE); KUESTERS,Esther, 40699 Erkrath (DE); SCHULTE, Petra, 50733 Köln (DE); STRAUSS, Gabriele, 40589 Düsseldorf (DE)
(74) Vertreter: BASF IP Association
(86) Internationale Anmeldenummer: PCT/EP2016/058504
(87) Internationale Veröffentlichungsnummer: WO 2016/173870

(56) Entgegenhaltungen:
- WO-A1-2007/091016
- US-A1- 2005 008 680
- Anonymous: "Cremophor RH Grades(Technical Information)", BASF , 1. Mai 2010 (2010-05-01), Seite 8PP, XP002744832, Gefunden im Internet: URL:http://dewolfchem.com/wp-content/uploa ds/2013/08/cremophor_rh_tds.pdf [gefunden am 2015-09-19]
- DATABASE GNPD [Online] MINTEL; 1. November 2010 (2010-11-01), Laboratorios Kinesia Puig: "Make-Up Remover Wipes", XP002744833, Database accession no. 1434456
- DATABASE GNPD [Online] MINTEL; 1. Februar 2010 (2010-02-01), Antonio Puig: "Make-Up Remover Wipes", XP002744834, Database accession no. 1263562

## Beschreibung

### Gebiet der Erfindung

Die Erfindung liegt auf dem Gebiet der ÖI-in-Wasser Emulsionen für die Kosmetik und betrifft niedrigviskose O/W-Emulsionen mit sehr kleinen Öl-Tröpfchengrößen, ein Verfahren zu deren Herstellung und deren Verwendung zur Imprägnierung von Textil und Papier für die Kosmetik.

### Stand der Technik

In der Kosmetik sind Öl-in-Wasser-Emulsionen (im folgenden O/W-Emulsionen) beliebt, die ein elegantes, feinteiliges Aussehen aufweisen, wie bläulich-schimmernde Emulsionen mit Öltröpfchengrößen unter 500 nm. Derartige Emulsionen können hergestellt werden nach dem sogenannten Phaseninversionsverfahren (PIT-Verfahren) durch Erwärmen von ausgewählten O/W-Emulsionen mit speziellen nichtionogenen Emulgatoren über der Phaseninversionstemperatur und anschließendem Abkühlen. Nach diesem PIT-Verfahren geht man von O/W-Emulsionen mit großen Öltröpfchen aus, die durch das Erwärmen eine Phasenumkehr in eine W/O-Emulsion erleiden, die reversibel ist, weshalb durch Abkühlung wieder der ursprüngliche O/W-Emulsionstyp hergestellt wird, die sog. PIT-Emulsion. Die so erhaltenen PIT-Emulsionen sind im Vergleich zu den eingesetzten O/W-Emulsionen besonders feinteilig und zeigen eine homogenere Öl-Tröpfchengrößenverteilung.

Aus der internationalen Anmeldung WO 89/11907 ist ein Verfahren zur Herstellung von niedrigviskosen PIT-Emulsionen bekannt, bei denen erstmals polare Triglyceride als Öle eingesetzt werden konnten unter Verwendung einer speziellen Emulgatormischung aus Fettalkoholethoxylaten oder Partialester von Fettsäuren mit HLB-Werten von 11 bis 12 und Co-Emulgatoren wie Fettalkoholen bzw. Partialester von Fettsäuren mit Polyolen.

Nach diesem Verfahren werden stabile PIT-Emulsionen erhalten, die sehr unterschiedliche Viskositäten im Bereich von 3 bis 1400 mPas bei 20 °C aufweisen. Des Weiteren sind Phaseninversionstemperaturen bis 95 °C notwendig. In der Praxis werden Phaseninversionstemperaturen von maximal 90 °C aufgrund des Wasseranteils in den Emulsionen gewünscht.

Aus der internationalen Anmeldung WO 01/89678 sind neue Emulgatoren für die Herstellung von PIT- oder Mikroemulsionen bekannt, die gegenüber dem Stand der Technik eine verbesserte Lagerstabilität ohne Vergelungen und Kristallisation bei hohen Temperaturen gewährleisten. Die dort vorgeschlagenen Emulgatoren enthalten Partialglyceride mit einem Monoglyceridanteil unter 50 Gew.-%, sowie Alkoholpolyglykolether und Fettalkohole. Als Co-Emulgatoren werden in einer langen Liste unter anderem auch Anlagerungsprodukte von 1 bis 15 Mol Ethylenoxid an Ricinusöl und/oder gehärtetem Ricinusöl sowie Anlagerungsprodukte von 15 bis 60 Mol Ethylenoxid an Ricinusöl und/oder gehärtetem Ricinusöl aufgelistet. Die beschriebenen Emulgatormischungen sind hervorragend geeignet für die Lagerung bei hohen Temperaturen, zeigen jedoch ein verbesserungsbedürftiges Verhalten bei der Lagerung in der Kälte.

Emulsionen zur Avivage von Textilien und Papier enthaltend Anlagerungsprodukte von 7 Mol Ethylenoxid an gehärtetem Ricinusöl werden in der internationalen Anmeldung WO2008/067944 beschrieben. Die dort genannten Emulsionen sind vom Typ der W/O- Emulsionen und zudem milchig-weiß bzw. milchig-gelb und weisen somit keine feinteilige, homogen verteilte Öltröpfchen auf.

Aufgabe der vorliegenden Erfindung war es, neue O/W-Emulsionen für die Kosmetik bereitzustellen, die
- sehr geringe Öltröpfchengrößen aufweisen, also sehr feinteilig sind
- eine homogene Verteilung der Öltröpfchen aufweisen, um eine homogene Verteilung der Öltröpchen bei der Anwendung, insbesondere deren Versprühung, zu bewirken
- sehr niedrigviskos sind, mit Viskositäten möglichst unter 300 mPas, um eine gute Versprühbarkeit zu gewährleisten
- auch unter schwankenden Temperaturbelastungen lagerstabil sind
- das gewünschte Verhalten wie Öltröpfchengrößen, Viskositäten insbesondere auch noch nach längerer Lagerung in der Kälte aufweisen
- problemlos mit Wasser verdünnbar sind, ohne dass es zu Vergelungen kommt oder zum Brechen der Emulsion
- bei Raumtemperatur mit Wasser verdünnbar sind, damit der Kunde nicht die Emulsion erwärmen muss,
- nach dem PIT-Verfahren herstellbar sind bei Temperaturen, die möglichst unter 90 °C, vorzugsweise unter 80 °C liegen
- für verschiedene Öle, besonders für polare Öle geeignet sind.

Überraschenderweise wurde gefunden, dass O/W-Emulsionen mit einer speziellen Emulgatormischung aus verschieden ethoxylierten hydrierten Ricinusölen in einem ganz ausgewählten Mengenverhältnis die komplexe Aufgabe lösen.

### Beschreibung der Erfindung

Ein Gegenstand der vorliegenden Erfindung betrifft niedrigviskose O/W-Emulsionen mit Öl-Teilchendurchmesser von 0,01 bis 0,3 µm für kosmetische Anwendungen bestehend aus
(a) einem oder mehreren Öl(en)
(b) einer Emulgatormischung
(c) Wasser
(d) und ggf. kosmetischen Wirk- und/oder Hilfsstoffen,
dadurch gekennzeichnet, dass die Emulgatormischung besteht aus
b1) Anlagerungsprodukten von 1 bis 15 Mol Ethylenoxid an hydriertem Ricinusöl und
b2) Anlagerungsprodukten von 20 bis 80 Mol Ethylenoxid an hydriertem Ricinusöl
in einem Masseverhältnis (= Emulgatorquotient) δ = Masse b2 :Masse (b1+ b2) im Bereich von 0,3 bis 0,8 und wobei die Öl(e) a) einen Polaritätsindex von 15 bis 55 [mN/m] aufweisen.

Weitere Gegenstände der vorliegenden Erfindung betreffen ein Verfahren zu deren Herstellung sowie die Verwendung zur Imprägnierung von Textil und Papier für die Kosmetik, insbesondere von Wet Wipes.

Im Sinne der vorliegenden Erfindung wird der Begriff "Öl" für Verbindungen gebraucht, die bei Zimmertemperatur (21 °C) flüssig und mit Wasser praktisch nicht mischbar sind.

Der Begriff "niedrigviskos" steht in der vorliegenden Anmeldung für Verbindungen, deren Viskositäten gemessen nach Brookfield/RVT/23°C+/-3°C/Sp 3/50rpm unter 300mPas liegen. Vorzugsweise weisen sie Viskositäten im Bereich von 1 bis 200 mPas bestimmt gemäß Brookfield/ RVT/23°C+/-3°C/Sp 3/50rpm auf.

Die Begriffe "Öl-Teilchendurchmesser bzw. Öl-Tröpfchendurchmesser" oder einfach nur Teilchen- bzw. Tröpfchendurchmesser "werden in der vorliegenden Anmeldung synonym gebraucht und beschreiben den Durchmesser der Öltröpfchen in [µm] in der O/W-Emulsion, bestimmt als Medianwert der Volumenverteilung mit dem Gerät: Beckman Coulter LS-230, Small Volume Modul; Messinfos / Parameter: Mie-Streuung, mit PIDS Daten, Brechungsindex von Wasser: 1,332, mit Brechungsindex 1,47 für die Ölphase. Die Öl-Teilchendurchmesser der erfindungsgemäßen O/W-Emulsionen liegen im Bereich von 0,01 bis 0,3 µm, vorzugsweise im Bereich von 0,06 bis 0,2 µm.

Der Polaritätsindex wird in der Einheit [mN/m] angegeben und ist ein Maß für die Polarität eines Öls definiert als Grenzflächenspannung des Öls gegen Wasser und wurde bestimmt unter Einsatz eines Ringtensiometers (Krüss K 10), der die Grenzflächenspannung in Analogie der ASTM Methode D971-99a, 2004, misst. In der Regel werden Öle mit Polaritäten über 35 mN/m als unpolar und zwischen 5 und 30 mN/m als polar bezeichnet.

Das Massenverhältnis der Emulgatoren wird in der für den Fachmann auf diesem Gebiet typischen Art und Weise als Emulgatorquotient δ angegeben und ist eine rechnerische Größe ermittelt aus dem Verhältnis der eingesetzten Massen m (berechnet als Aktivsubstanzgehalt) an Emulgatoren nach der allgemeinen Beziehung δ= Masse Emulgator 1 : (Masse Emulgator 1 + Masse Emulgator 2).

### Öle

Im Sinne der vorliegenden Erfindung können als Komponente a) der erfindungsgemäßen O/W-Emulsion ein oder mehrere Öle vorliegen, d.h. es kann sich um ein einziges Öl oder auch um Mischungen verschiedener Öle handeln. Die Öle können polar und/oder unpolar sein.

Beispielsweise sind die nachstehend genannten Verbindungsklassen geeignet: Guerbetalkohole auf Basis von Fettalkoholen mit 6 bis 18, vorzugsweise 8 bis 10 Kohlenstoffatomen, z.B. 2-Ethylhexanol oder 2-Octyldodecanol; Ester von linearen oder verzweigten, gesättigten oder ungesättigten C₆-C₂₄-Fettsäuren mit linearen oder verzweigten, gesättigten oder ungesättigten C₆-C₂₄-Fettalkoholen. Beispielhaft seien genannt Hexyllaurat, Ethylhexylstearat, Myristylisostearat, Myristyloleat, Cetearylisononanoat, Cetylisostearat, Cetyloleat, Decyloleat, Stearylisostearat, Stearyloleat, Isopropylmyristat, Isostearylmyristat, Isostearylpalmitat, Isostearylstearat, Isostearylisostearat, Isostearyloleat, Oleylmyristat, Oleylisostearat, Oleyloleat, Oleylerucat, Erucylisostearat, Erucyloleat, Cococaprylat/caprat. Weitere geeignete Ester sind z.B. Ester von C₁₈-C₃₈-Al-kylhydroxycarbonsäuren mit linearen oder verzweigten, gesättigten oder ungesättigten C₆-C₂₂-Fettalkoholen, Ester von linearen und/oder verzweigten, gesättigten oder ungesättigten Fettsäuren mit mehrwertigen Alkoholen (wie z. B. Propylenglycol, Dimerdiol oder Trimertriol) und/oder Guerbetalkoholen, Triglyceride oder Triglyceridmischungen, flüssige Mono-/Di-/Triglyceridmischungen, Ester von C₆-C₂₂-Fettalkoholen und/oder Guerbetalkoholen mit aromatischen Carbonsäuren, insbesondere Benzoesäure (z. B. Finsolv® TN), Ester von C₂-C₁₂-Dicarbonsäuren mit linearen oder verzweigten, gesättigten oder ungesättigten Alkoholen mit 1 bis 22 Kohlenstoffatomen oder Polyolen mit 2 bis 10 Kohlenstoffatomen und 2 bis 6 Hydroxylgruppen. Geeignet sind auch pflanzliche Öle, Triglyceridmischungen, substituierte Cyclohexane, lineare symmetrische oder unsymmetrische Dialkylcarbonate (z.B. Cetiol® CC), Guerbetcarbonate auf Basis von Fettalkoholen mit 6 bis 18, vorzugsweise 8 bis 10 C-Atomen, lineare oder verzweigte, symmetrische oder unsymmetrische Dialkylether mit 6 bis 22 Kohlenstoffatomen pro Alkylgruppe, wie z. B. Din-octyl Ether (Cetiol® OE), Ringöffnungsprodukte von epoxidierten Fettsäureestern mit Polyolen, Kohlenwasserstoffe wie Paraffin- oder Mineralöle, Oligo- oder Polyalphaolefine. Die Dialkylcarbonate und Dialkylether können symmetrisch oder unsymmetrisch, verzweigt oder unverzweigt, gesättigt oder ungesättigt sein und lassen sich nach Reaktionen, die aus dem Stand der Technik hinlänglich bekannt sind, herstellen. Geeignete Siliconverbindungen sind beispielsweise Dimethylpolysiloxane, Methylphenylpolysiloxane, cyclische Silicone (Cyclomethicon) sowie amino-, fettsäure-, alkohol-, polyether-, epoxy-, fluor-, glykosid- und/oder alkylmodifizierte Siliconverbindungen. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

Im Sinne der vorliegenden Erfindung weisen die Öl(e) a) einen Polaritätsindex von 15 bis 55 [mN/m] auf.

Einer bevorzugten Ausführungsform entsprechend sind polare Öle geeignet, die einen Polaritätsindex von 15 bis 30 mN/m aufweisen. Beispiele für geeignete polare Öle sind Mono-, Di und Trifettsäureester von Glycerin, die vorzugsweise 6 bis 24 und insbesondere 8 bis 18 Kohlenwasserstoffatome aufweisen und gesättigt und/oder ungesättigt sein können und durch chemische Synthese aus einer natürlichen (pflanzlichen oder tierischen) Quelle erhalten werden. Ein Beispiel ist ein Triglyceridester einer C8/C10 Fettsäuremischung, der unter dem Handelsnamen Myritol ®312 von der BASF Personal Care & Nutrition GmbH erhältlich ist.

Weitere geeignete polare Öle sind die in der kosmetischen Industrie weithin bekannten pflanzlichen Öle wie Erdnussöl, Kokosnussöl, Maisöl, Olivenöl, Palmkernöl, Sonnenblumenöl, Sojaöl, Rapsöl, Mandelöl, Traubenkernöl, Distelöl, Nachtkerzenöl, Macadamianussöl, Arganöl, Avocadoöl und dergleichen mehr.

Weiterhin sind als polare Öle die Ester der Benzoesäure geeignet, vorzugsweise die Benzoesäureester von Alkoholen mit 8 bis 22 Kohlenstoffatomen, und insbesondere die Benzoesäureester von geradkettigen Alkoholen mit 12 bis 15 Kohlenstoffatomen. Hervorragend geeignet sind Benzoesäureester einer C12-C15 Alkoholmischung, die beispielsweise unter dem Handelsnamen Cetiol® AB (INCI: C12-C15 Alkyl Benzoate) von der BASF Personal Care & Nutrition GmbH erhältlich sind.

Ebenso eignen sich als polare Öle die sogenannten Esteröle, d.h. Ester von linearen C6-C22 Fettsäuren mit linearen oder verzweigten C6-C22- Fettalkoholen bzw. Ester von verzweigten C3-C13-Carbonsäuren mit linearen oder verzweigten C6-C22-Fettalkoholen bzw. Ester von linearen C6-C22-Fettsäuren mit verzweigten C3-C13-Alkoholen. Als Beispiele seien genannt Decyloleat, 2-Ethylhexylpalmitat, Isopropylstearat, Isopropylmyristat sowie Cetylstearyloctanoat.

Als Öle mittlerer Polarität mit einem Polaritätsindex, der in der Regel über 30 bis etwa 35 mN/m liegt, sind Dialkylcarbonate vorteilhaft, die sich vorzugsweise von Fettsäuren mit 6 bis 12 Kohlenstoffatomen ableiten wie Dicaprylylcarbonat.

Als unpolare Öle mit einem Polaritätsindex von etwa über 35 mN/m können die weithin aus der kosmetischen Industrie bekannten Öle eingesetzt werden, vorzugsweise Kohlenwasserstoff basierte Öle wie aliphatische und/oder aromatische Öle, die vorzugsweise 8 bis 32, insbesondere 15 bis 20 Kohlenstoffatome aufweisen. Beispiele sind Squalan, Squalen, Paraffinöle, Isohexadecan, Isoeicosecan, Polyolefine wie Polydecene, hydrogenierte Polyisobutene, Dialkylcyclohexan und Mineralöl. Bevorzugt sind Paraffinöle wie die dünnflüssigen Paraffine (*Paraffinum per*/*iquidum*), die eine Viskosität von 25 bis 80 mPa·s haben und/oder die dickflüssigen Paraffine (*Paraffinum subliquidum*)*,* die als ölige Flüssigkeit eine Viskosität von 110 bis 230 mPa·s aufweisen. Weiterhin bevorzugt sind die hydrogenierten Polyisobutene, die beispielsweise unter dem Handelsnamen Luvitol® Lite von der BASF SE erhältlich sind.

Ganz besonders bevorzugt im Sinne der Erfindung werden Öle, die ausgewählt sind aus der von Triglyceriden von C6-C22 Fettsäuren, hydrierten Polyisobutenen, Benzoesäureester von Alkoholen mit 8 bis 22 Kohlenstoffatomen und Ester von C6-C22 Fettsäuren mit linearen C6-C22 Fettalkoholen gebildeten Gruppe. Insbesondere geeignet sind hydrierte Polyisobutene, Benzoesäureester von Alkoholen mit 8 bis 22 Kohlenstoffatomen, Triglyceride von C6-C12 Fettsäuren und Ester von C6-C12 Fettsäuren mit linearen ungesättigten C6-C22 Fettalkoholen gebildeten Gruppe. Ganz besonders hervorzuheben sind im Sinne der Erfindung als Öle Benzoesäureester von Alkoholen mit 8 bis 22 Kohlenstoffatomen, und insbesondere Benzoesäureester von einem C12-C15 Alkoholgemisch.

In der folgenden Tabelle sind die Polaritäten der typischen, meist genutzten Öle aufgeführt. Weitere Öle mit deren Polaritätsindices sind in der DE 102004003436 A1 beschrieben, die hier miteinbezogen werden:

| **Öl** | **Polaritäts Index [mN/m]** |
|---|---|
| Isoparaffin (C₁₂-C₁₄) | 53.0 |
| Squalan® | 46.2 |
| Isohexadekan (ARLAMOL® ND) | 43.8 |
| Mineral Öl (Paraffin oil perliquidum) | 43.7 |
| Mineral Öl (Paraffin oil subliquidum) | 38.3 |
| Hydrogenated Polylsobutene (Luvitol® Lite) | 44.7 |
| Isoeikosan | 41.9 |
| Dioctylcyclohexane | 39.0 |
| Cetystearyloctanoat | 28,6 |
| Isopropylmyristat | 24,2 |
| 2-Ethylhexylpalmitat | 23,1 |
| Isopropylstearat | 21,9 |
| Decyloleat | 18,7 |
| C12-C15 Alkyl Benzoate | 17.1 |
| Rapsöl | 21.9 |
| Capryl/Caprin-Triglyceride | 21.3 |
| Erdnussöl | 20.5 |
| Mandelöl | 20.3 |
| Sonnenblumenöl | 19.3 |
| Avocadoöl | 18.3 |
| Olivenöl | 16.9 |

### Emulgatoren

Im Sinne der vorliegenden Erfindung ist es wesentlich, als Emulgatoren ausschließlich die Mischung der Emulgatoren b1) und b2) einzusetzen. Es handelt sich um Anlagerungsprodukte von Ethylenoxid an hydriertem Ricinusöl. Ricinusöl wird aus den Samen des Wunderbaums gewonnen und besteht hauptsächlich aus Triglyceriden der Ricinolsäure, auch Triricinolein genannt, und anderen Fettsäuren sowie einigen flüchtigen Bestandteilen. In der Regel liegt folgender Fettsäureanteil in den Triglyceriden vor:
77-83 Gew.-% Ricinolsäure
3-5 Gew.-% Linolsäure
4-9 Gew.-% Ölsäure
1-2 Gew.-% Palmitinsäure
1-3 Gew.-% Stearinsäure
sowie untergeordnete Mengen an weiteren, anderen Fettsäuren.

Bei hydriertem Ricinusöl wurden die Doppelbindungen der ungesättigten Fettsäuren hydriert. Ein Maß für die Vollständigkeit der Hydrierung ist die Jodzahl, die vorzugsweise unter 2,0 g/100 g nach ISO 3961 liegt. Derartiges hydriertes Ricinusöl ist dem Fachmann bekannt und im Handel erhältlich.

Emulgator b1) und b2) sind Anlagerungsprodukte von Ethylenoxid an hydriertem Ricinusöl, wobei die Anlagerung von Ethylenoxid, wie dem Fachmann bekannt ist, einer statistischen Verteilung entspricht und somit einen Durchschnittswert angibt.

Bei Emulgator b1) handelt es sich um Anlagerungsprodukte von 1 bis 15 Mol Ethylenoxid an hydriertem Ricinusöl, bevorzugt sind als Emulgatoren b1) Anlagerungsprodukte von 3 bis 12 Mol, besonders bevorzugt 5 bis 10 Mol und insbesondere 7 Mol Ethylenoxid an hydriertem Ricinusöl. Das mit 7 Mol Ethylenoxid an hydriertem Ricinusöl angelagerte Produkt mit dem INCI-Namen PEG-7 Hydrogenated Castor Oil ist unter dem Namen Cremophor® WO 7 Surfactant von der BASF erhältlich.

Bei Emulgator b2) handelt es sich um Anlagerungsprodukte von 20 bis 80 Mol Ethylenoxid an hydriertem Ricinusöl, ebenfalls mit statistischer Verteilung von Ethylenoxid. Bevorzugt sind als Emulgatoren b2) Anlagerungsprodukte von 25 bis 60 Mol, besonders bevorzugt 35 bis 60 Mol und insbesondere 38 bis 45 Mol Ethylenoxid an hydriertem Ricinusöl. Das mit 40 Mol Ethylenoxid an hydriertem Ricinusöl angelagerte Produkt mit dem INCI-Namen PEG-40 Hydrogenated Castor Oil ist unter dem Namen Eumulgin® CO 40 von der BASF erhältlich und außerordentlich hervorragend geeignet.

Zusätzlich ist im Sinne der Erfindung das Massenverhältnis von b2 : b1 wesentlich, das typischerweise vom Fachmann als Emulgatorquotient δ= Masse b2 : Masse (b1+b2) angegeben wird und im Bereich von 0,3 bis 0,8 liegt.

Die Öle a) und die Emulgatormischung b) liegen vorzugsweise in einem Gewichtsverhältnis von 1:3 bis 3:1, besonders bevorzugt 1:2 bis 2:1 und insbesondere 1:1,5 bis 1,5:1 vor.

Einer Ausführungsform der vorliegenden Erfindung entsprechend zeigen O/W-Emulsionen mit hydrierten Polyisobutenen als a) und einer Emulgatormischung b) aus b1) Anlagerungsprodukten von 7 Mol Ethylenoxid an hydriertem Ricinusöl und b2) Anlagerungsprodukten von 38 bis 45 Mol Ethylenoxid an hydriertem Ricinusöl mit einem Emulgatorquotient δ= Masse b2) : ((b1) + (b2)) im Bereich von 0,5 bis 0,6 sehr gute Eigenschaften.

Einer weiteren Ausführungsform der vorliegenden Erfindung entsprechend sind O/W-Emulsionen sehr vorteilhaft, die Benzoesäureester von Alkoholen mit 8 bis 22 Kohlenstoffatomen als Öl a) und eine Emulgatormischung b) aus b1) Anlagerungsprodukten von 7 Mol Ethylenoxid an hydriertem Ricinusöl und b2) Anlagerungsprodukten von 38 bis 45 Mol Ethylenoxid an hydriertem Ricinusöl mit einem Emulgatorquotient δ im Bereich von 0,3 bis 0,8 enthalten. Es handelt sich hierbei um besonders feinteilige O/W-Emulsionen mit extrem kleinen Öl-Tröpfchendurchmesser. Als weiteren zwingenden Bestandteil enthalten die O/W-Emulsionen Wasser, wobei die Menge an Wasser ad 100 Gew.-% beträgt und in der Regel zwischen 40 bis 80 Gew.-% liegt.

### Kosmetische Wirk- und Hilfsstoffe

Kosmetische Wirkstoffe und Hilfsstoffe sind fakultativ und richten sich nach dem kosmetischen Anwendungszweck der erfindungsgemäßen O/W-Emulsionen.

Typischerweise sind zumindest Hilfsstoffe wie Konservierungsmittel und/oder pH-Regulantien zugegen.

Als Konservierungsmittel sind Benzoesäure oder deren Salze, Phenoxyethanol, Ethylhexlglycerin, Dicaprylyl Glycol, Formaldehydlösung, Parabene, Pentandiol, Sorbinsäure, Dehydracetsäure und/oder die unter der Bezeichnung Surfacine® bekannten Silberkomplexe sowie die in Anlage 6, Teil A und B der Kosmetikverordnung aufgeführten weiteren Stoffklassen geeignet. Als pH-Wert Regulantien können die dem Fachmann für Kosmetik bekannten Verbindungen eingesetzt werden, beispielsweise Citronensäure bzw. deren Salze wie Trinatriumcitrat, Milchsäure bzw. deren Salze, Gluconsäure bzw. deren Salze wie Natriumgluconat.

Die pH-Wert Regulantien werden dabei vorzugsweise in solchen Mengen eingesetzt, dass der pH-Wert der O/W-Emulsionen im Bereich von 3 bis 7, vorzugsweise von 3,5 bis 5,5 liegt. Puffersysteme sind hierbei besonders beliebt.

Des Weiteren sind häufig auch wasserlösliche Verdickungsmittel wie natürliche und/oder synthetische Polymere wie Xanthan-Gum, Hydroxyethylcellulose, Polyvinylpyrrolidon oder hochmolekulare Polyethylenoxide als Hilfsstoffe enthalten.

Auch Hydrotrope können zur Verbesserung des Fließverhaltens enthalten sein. Typische Beispiele sind Glycerin; Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin und/oder Dialkoholamine, wie Diethanolamin oder 2-Amino-1,3-propandiol.

Gerne sind ebenfalls Parfümöle als kosmetischer Wirkstoff und/oder Hilfsstoff zugegen. Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/ Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmeth-acrylat/tert.Butyl-aminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in Cosm.Toil. 108, 95 (1993**)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976**).**

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

### Insekten-Repellentien

Als Insekten-Repellentien kommen N,N-Diethyl-m-toluamid, 1,2-Pentandiol oder Ethyl Butylacetylaminopropionate in Frage.

Ganz besonders bevorzugt enthalten die erfindungsgemäßen O/W-Emulsionen kosmetische Wirk- und/oder Hilfsstoffe und bestehen insbesondere aus
15 bis 22 Gew.-% Benzoesäureester von Alkoholen mit 8 bis 22 Kohlenstoffatomen
18 bis 26 Gew.-% einer Emulgatormischung b) aus
b1) Anlagerungsprodukte von 7 Mol Ethylenoxid an hydriertem Ricinusöl und
b2) Anlagerungsprodukte von 38 bis 45 Mol Ethylenoxid an hydriertem Ricinusöl mit einem Emulgatorquotient δ im Bereich von 0,3 bis 0,8, und
47 bis 76,5 Gew.-% Wasser und
0,2 bis 5 Gew.-% kosmetische Wirk- und Hilfsstoffe.

Vorteilhafterweise sind in den erfindungsgemäßen O/W-Emulsionen als Hilfs-und/oder Wirkstoffe d) Konservierungsmittel und/oder pH-Regulantien zugegen.

Die erfindungsgemäßen O/W-Emulsionen mit ihren besonders kleinen Öltröpfchendurchmesser und feinen Verteilungen werden vorzugsweise nach dem Phaseninversionsverfahren hergestellt.

Ein weiterer Gegenstand betrifft daher ein Verfahren zur Herstellung von niedrigviskosen O/W-Emulsionen mit Öl-Teilchendurchmesser von 0,01 bis 0,3 µm nach Anspruch 1 nach dem Phaseninversionsverfahren, dadurch gekennzeichnet, dass eine O/W-Emulsion mit Teilchendurchmesser über 2 µm bestehend aus
(a) einem oder mehreren Öl(en) mit einem Polaritätsindex von 15 bis 55 [mN/m],
(b) einer Emulgatormischung bestehend aus
b1) Anlagerungsprodukten von 1 bis 15 Mol Ethylenoxid an hydriertem Ricinusöl und
b2) Anlagerungsprodukten von 20 bis 60 Mol Ethylenoxid an hydriertem Ricinusöl in einem Masseverhältnis δ= Masse b2) : ((b1) + (b2)) im Bereich von 0,3 bis 0,8
(c) Wasser
(d) und ggf. kosmetischen Wirk- und/oder Hilfsstoffen,
einsetzt, auf Temperaturen im Bereich von 40 °C bis 90 °C erhitzt und anschließend auf Raumtemperatur abgekühlt.

Vorzugsweise erfolgt das Verfahren bei Temperaturen von 60 bis 90 °C. Innerhalb dieses Temperaturbereiches liegt die sogenannte PIT-Temperatur, also der Temperaturbereich, auf den die im 1. Verfahrensschritt hergestellte grobteilige O/W-Emulsion im 2. Verfahrensschritt erwärmt werden muss, damit eine Phaseninversion in eine W/O-Emulsion erfolgt. Dieser Temperaturbereich kann durch Verfolgung der elektrischen Leitfähigkeit der Emulsion in Abhängigkeit der Temperatur unter Verwendung einer 4-Elektroden-Leitfähigkeitsmesszelle Tetracon 325/S der Firma WTW ermittelt werden. Der Temperaturbereich, innerhalb welchem die Leitfähigkeit der im 1. Verfahrensschritt hergestellten O/W-Emulsionen auf Werte unter 0,1 mS/cm abfällt, ist die PIT-Temperatur.

Im Sinne der Erfindung wird die im 2. Verfahrensschritt hergestellte invertierte W/O-Emulsion abgekühlt, vorzugsweise mit Abkühlraten von 0,5 bis 2,5 °C pro Minute. Hierbei erfolgt erneut eine Phasenumkehr und man erhält die besonders feinteiligen, niedrigviskosen, lagerstabilen erfindungsgemäßen O/W-Emulsionen mit den vielen vorteilhaften Eigenschaften der oben beschriebenen Art.

### Gewerbliche Anwendbarkeit

Praktisch bedeutsam ist die unkomplizierte Art der Verdünnbarkeit der erfindungsgemäßen O/W-Emulsionen mit Wasser. So ist es möglich, ohne ungewünschte Gelphasen durchlaufen zu müssen, beliebig konzentrierte Verdünnungen der O/W-Emulsionen herzustellen, die allesamt eine niedrige Viskosität aufweisen und sich daher hervorragend auf verschiedene Oberflächen applizieren lassen.

Ein weiterer Gegenstand der vorliegenden Erfindung betrifft daher die Verwendung der O/W-Emulsionen der beschriebenen Art zur Imprägnierung von Textil und Papier für die Kosmetik, insbesondere von Wet Wipes.

Bevorzugt werden die erfindungsgemäßen O/W-Emulsionen in Form ihrer wässrigen Verdünnungen verwendet, wobei die O/W-Emulsionen vorzugsweise in Mengen von 0,2 bis 10 Gew.-% - bezogen auf wässrige Verdünnung - enthalten sind.

Unter dem Oberbegriff "Textil und Papier" werden verschiedene Sorten und Artikel verstanden, die sich in ihren Anwendungsgebieten und ihrer Beschaffenheit zum Teil erheblich unterscheiden können. Geeignet sind beispielsweise Tissuepapiere und/oder Tissuegewebe und/oder Tissuetücher (im Weiteren mit Tissuetücher bezeichnet). Diese können ein- oder mehrlagig aufgebaut sein. In der Regel weisen die Papiere ein Quadratmetergewicht von 10 bis 65, vorzugsweise 15 bis 30 g und eine Dichte von 0,6 g/cm und weniger auf. Beispiele für Tissuepapiere sind Toilettenpapiere, Papiertaschentücher, Gesichtsreinigungstücher, Abschminktücher, Erfrischungstücher, Haushaltstücher und dergleichen. Neben den papierbasierten Tissues kommen auch entsprechende Tissuegewebe in Frage, die aus Faser- oder Fleecestoff hergestellt werden.

Bevorzugt sind mehrlagige Tissuetücher. Insbesondere sind solche Tissuetücher bevorzugt, welche zwischen den einzelnen Lagen eine undurchlässige und/oder teildurchlässige Sperrschicht haben. Die teildurchlässige Sperrschicht kann beispielsweise als semipermeable Membran ausgebildet sein. Mit derartigen Tüchern können zwei oder mehrere Zusammensetzungen (gegebenenfalls nach vorheriger Verdünnung) auf ein Tuch aufgebracht werden. Dies kann ganz besonders bevorzugt sein, um mit der einen Seite der Tücher die Reinigung mittels der auf das Tuch aufgebrachten Zusammensetzung zu bewirken. Mit der anderen Seite kann dann beispielsweise zum Trocknen nachgerieben werden oder gegebenenfalls ein pflegender Wirkstoff auf die Haut aufgetragen werden.

Weiterhin können die Tücher aus mindestens 3 Lagen mit Zusammensetzungen (gegebenenfalls nach vorheriger Verdünnung) behandelten Tissuetuches bestehen. Vorteilhaft ist dann zwischen mindestens 2 Lagen behandeltem Tuch jeweils 1 Lage Tuch als semipermeable Membran ausgebildet. Die semipermeable Membran ist dabei in Richtung der äußeren Tuchlagen durchlässig. Dadurch kann im Inneren beispielsweise eine Zusammensetzung (gegebenenfalls nach vorheriger Verdünnung) auf die innerste Schicht aufgebracht werden, welche entweder nicht mischbar und/oder nicht stabil mit der auf die äußere Seite aufgetragenen Zusammensetzung ist. Hierdurch wird es möglich "two in one Tücher" zur Reinigung und Pflege anzubieten. Die unterschiedliche farbliche Gestaltung der Tuchlagen sowie der unterschiedliche Aufbau der Tücher aus mehreren Materialien, insbesondere in Bezug auf die Saugfähigkeit und Durchlässigkeit der unterschiedlichen Tuchlagen, ist ebenso möglich.

Weiterhin eignen sich beispielsweise Textilfasern sowohl aus Naturfasern wie z.B. Cellulose, Seide, Wolle, Regeneratcellulose (Viskose, Rayon), Cellulosederivate als auch Textilfasern aus synthetischen Fasern wie z.B. Polyester-, Polypropylen-, Polyethylenterephtalat-, Polyamid-, Polyolefin-, Polyacrylnitril-Fasern oder Mischungen solcher Fasern. Diese Fasern können gewebt oder ungewebt sein.

Der Begriff "Imprägnieren" umfasst dabei jede Art der Aufbringung der erfindungsgemäßen O/W-Emulsion bzw. eine seiner wässrigen Verdünnungen auf mindestens eine Seite des Textils bzw. Papiers. Hierzu eignen sich alle einschlägig bekannten Methoden, mit deren Hilfe man Flüssigkeiten auf mehr oder weniger feste Oberflächen auftragen kann. Beispielsweise seien genannt: Tränken, Beschichten, Be- oder Ansprühen, Tauchen, Ausrüsten, Abstreifen etc. Die Imprägnierung kann dabei bei Raumtemperatur oder unter Hitzeeinwirkung vorgenommen werden. Nach dem Auftragen der O/W-Emulsionen bzw. deren wässrigen Verdünnungen kann sich ein kurzer Trockenschritt anschließen.

Vorzugsweise werden die erfindungsgemäßen O/W-Emulsionen in Form einer wässrigen Verdünnung verwendet, wobei je nach dem gewünschten Anwendungszweck weitere, zusätzliche kosmetische Wirk- und/oder Hilfsstoffe enthalten sind. Dabei kann es sich um die gleichen kosmetischen Wirk- und/oder Hilfsstoffe handeln, die bereits unter d) im Zusammenhang mit den erfindungsgemäßen O/W-Emulsionen beschrieben worden sind oder auch um weitere übliche, dem Fachmann bekannten Wirk- und Hilfsstoffe (e), die in wässrigen Verdünnungen auf Textil und Papier aufgebracht werden und insbesondere als Wet Wipes in den Handel kommen. Somit sind die unter d) beschriebenen Hilfs-und/oder Wirkstoffe entweder in den erfindungsgemäßen O/W-Emulsionen enthalten oder werden den wässrigen Verdünnungen zugegeben. Die weiteren, zusätzlichen kosmetischen Hilfs- und Wirkstoffe (e) sind im Unterschied zu d) erst in den Verdünnungen enthalten.

Exemplarisch für die zusätzlichen Hilfs- und Wirkstoffe e) sind hier Tenside, Selbstbräuner, UV-Filter, Solubilisatoren, Feuchthaltemittel bzw. Hautbefeuchtungsmittel oder auch weitere Öle beispielsweise die unter a) genannten Öle zu nennen.

So sind Tenside typischerweise in Formulierungen für Wet Wipes zur Reinigung der Haut, Öle für Wet Wipes zur Pflege und/oder Schmutzentfernung und UV-Filter bzw. Selbstbräuner für Wet Wipes zum Schutz vor UV-Strahlung oder zur Selbstbräunung zusätzlich als Wirk-und/oder Hilfsstoff enthalten.

### Tenside

Tenside im Sinne der Erfindung sind amphiphile Stoffe, die die Entfernung und Lösung von Schmutz, ein leichtes Abspülen des Schmutzes und - je nach Wunsch - Schaumregulierung bewirken.

Als Tenside können anionische, nichtionische, kationische und/oder amphotere bzw. zwitterionische Tenside enthalten sein. In tensidhaltigen kosmetischen Zubereitungen ist vorzugsweise wenigstens ein anionisches Tensid enthalten.

Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als zwitterionische Tenside werden solche oberflächenaktiven Verbindungen bezeichnet, die im Molekül mindestens eine quartäre Ammoniumgruppe und mindestens eine -COO(-)- oder -SO3(-)-Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C8-C18-Alkyl- oder Acylgruppe im Molekül mindestens eine freie Aminogruppe und mindestens eine -COOH- oder -SO3H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12-18-Acylsarcosin.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/oder deren Gemische mit Alkyloligoglucosidcarboxylaten, Fettsäureglucamide, Alkylamidobetaine, Ampho-acetate und/oder Proteinfettsäurekondensate, letztere vorzugsweise auf Basis von Weizenproteinen bzw. deren Salzen.

**Anionische Tenside** sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methylhydroxyalkyldialkoyloxyalkylammoniummethosulfate und die entsprechenden Produkte der Dehyquart®-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

Die derart imprägnierten Textile und Papiere in der Kosmetik finden vorzugsweise im Bereich der Wet Wipes zur Hautpflege oder -reinigung (insbesondere der Babypflege oder -reinigung) ihre Anwendung. Beispielsweise seien genannt Pflege- oder Reinigungstücher für die Gesichtshaut (sog. Facial tissues, Abschminktücher/Make-up-Entfernung etc.), Erfrischungstücherfür die Haut, antibakterielle und/oder desodorierende Tücher, Produkte für die Intimpflege; wie beispielsweise Tampons, Damenbinden, Slipeinlagen, Intimpflegetücher), feuchtes Toilettenpapier, Inkontinenzprodukte, Selbstbräunungstücher oder sog. Insect Repellent Tücher.

Für Textile und Papiere in der Kosmetik, die dem Sonnenschutz oder der Selbstbräunung dienen, sind vorzugsweise in den erfindungsgemäßen, mit Wasser verdünnten O/W-Emulsionen eine oder mehrere UV-Lichtschutzfilter oder Selbstbräuner enthalten.

Als **UV-Lichtschutzfilter** sind bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
3-(4'-Trimethylammonium) benzyliden- bornan-2-on-methylsulfat (Mexoryl SO)
3,3'-(1,4-Phenylendimethin)-bis (7,7-dimethyl-2-oxobicyclo-[2.2.1]heptan-1-methansulfonsäure) and salts (Mexoryl SX)
3-(4'-Sulfo)-benzyliden-bornan-2-on and salts (Mexoryl SL) Polymer von N-[2(und 4)-(2-oxoborn-3-ylidenmethyl)benzyl]acrylamid Mexoryl SW)
2-(2H-Benzotriazol-2-yl)-4-methyl-6-(2-methyl-3-(1,3,3,3-tetramethyl-1-(trimethylsilyloxy)disiloxanyl)propyl)phenol (Mexoryl XL)
4-Aminobenzoesäurederivate, vorzugsweise 4-(Dimethylamino)benzoesäure-2-ethyl-hexylester,
4-(Dimethylamino)benzoesäure-2-octylester und 4-(Dimethylamino)benzoe-säureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
Ester der Salicylsäure, vorzugsweise Salicylsäure-2-ethylhexylester, Salicylsäure-4-iso-propylbenzylester, Salicylsäurehomomenthylester;
Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
Ester der Benzalmalonsäure, vorzugsweise 4-Methoxybenzmalonsäuredi-2-ethylhexyl-ester;
Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl)anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4-diyl)diimino]bis(benzoesäure-2-ethylhexylester) (Uvasorb® HEB);
2,2(-Methylen-bis(6-(2H-benzotriazol-2-yl)-4-(1,1,3,3-tetramethyl-butyl)phenol) (Tinosorb M);
2,4-Bis[4-(2-ethylhexyloxy)-2-hydroxyphenyl]-6-(4-methoxyphenyl)-1,3,5-triazin (Tinosorb S); Propan-1,3-dione, wie z.B. 1-(4-tert.Butylphenyl)-3-(4'methoxyphenyl)propan-1,3-dion;
Ketotricyclo(5.2.1.0)decan-Derivate, wie in der EP 0694521 B1 beschrieben;
Dimethicodiethylbenzalmalonate (Parsol SLX).

Als wasserlösliche UV - Filter kommen in Frage:
2-Phenylbenzimidazol-5-sulfonsäure und deren Alkali-, Erdalkali-, Ammonium-, Alkylammonium-, Alkanolammonium- und Glucammoniumsalze;
2,2(-(1,4-Phenylen)bis(1H-benzimidazol-4,6-disulfonsäure, Mononatriumsalz) (Neo Heliopan AP) Sulfonsäurederivate von Benzophenonen, vorzugsweise 2-Hydroxy-4-methoxybenzophenon-5-sulfonsäure und ihre Salze;
Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der Fassung **Commission Directive 2005/9/EC of 28 January 2005 amending Council Directive 76/768/EEC,** concerning cosmetic products, for the purposes of adapting Annexes VII thereof to technical progress) zugelassen Stoffe, auf die hier explizit Bezug genommen wird.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für Zinkoxide sind z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P. Finkel in SÖFW-Journal 122, 8/1996, S. 543-548 sowie Parf. Kosm. 80. Jahrgang, Nr. 3/1999, S. 10 bis 16 zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. alpha-Carotin, beta-Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Solubilisatoren

Typische Beispiele für Solubilisatoren sind INCI: Polysorbate 20 (Eumulgin® SML 20), INCI: Polysorbate 80 (Eumulgin® SMO 20), INCI: Polysorbate 60 (Eumulgin® SMS 20).

Des Weiteren können als Hilf-und/oder Wirkstoff Feuchthaltemittel bzw. Hautbefeuchtungsmittel zur Verbesserung der sensorischen Eigenschaften sowie zur Feuchtigkeitsregulierung der Haut enthalten sein. Es kann außerdem dazu beitragen, das Eindringvermögen der Zusammensetzung auf den Wipes zu verbessern.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin und Triglycerin.

Die erfindungsgemäßen, mit Wasser verdünnten O/W-Emulsionen enthalten vorzugsweise die zusätzlichen, weiteren kosmetischen Hilfs- und/oder Wirkstoffe d) und/oder e) in einer Menge von 0 bis 40 Gew.-%., vorzugsweise 0,05 bis 30 Gew.-%, insbesondere 0,05 bis 20 Gew.-%, vorzugsweise 0,1 bis 15 Gew.-% und insbesondere 0,1 bis 10 Gew.-%, bezogen auf das Gesamtgewicht der mit Wasser verdünnten O/W Emulsion.

Besonders bevorzugt werden die erfindungsgemäßen O/W-Emulsionen in Form ihrer wässrigen Verdünnungen zur Imprägnierung von Wet Wipes verwendet, also zu feuchten Textil- oder Papier-Produkten führen.

Ein weiterer Gegenstand der vorliegenden Erfindung betrifft daher Wet Wipes für die Kosmetik, imprägniert mit einer wässrigen Verdünnung einer erfindungsgemäßen O/W-Emulsion.

### Beispiele

Eingesetzt wurden folgende Handelsprodukte der BASF SE bzw. der BASF Personal Care & Nutrition GmbH:
Eumulgin® CO 60 = mit 60 Mol Ethylenoxid ethoxyliertes, hydriertes Ricinusöl; INCI : PEG-60 Hydrogenated Castor Oil
Eumulgin® CO 40 = mit 40 Mol Ethylenoxid ethoxyliertes, hydriertes Ricinusöl; INCI : PEG-40 Hydrogenated Castor Oil
Cremophor® WO 7 = mit 7 Mol Ethylenoxid ethoxyliertes, hydriertes Ricinusöl; INCI : PEG-7 Hydrogenated Castor Oil
Cetiol® AB = Benzoesäureester einer C12-15 Alkoholmischung; INCI : C12-15 Alkyl Benzoate
Luvitol® Lite = hydrogeniertes Polyisobutene gemäß Beispiel 3 der WO95/14647 unter Verwendung des in Beispiel 1 aus der WO95/14647 beschriebenen Katalysators, nach einer Fraktionierung erhaltene Fraktion 1c aus 7% C12-Oligomerisierungsprodukt, 70% C16 Oligomerisierungsprodukt und 17% C20 Oligomerisierungsprodukt sowie höheren Homologen gemäß GC-Analyse; INCI : Hydrogenated Polyisobutene
Myritol® 312= INCI: Caprylic/Capric Triglyceride
Cetiol® V = INCI : Decyl Oleate

### Beispiele 1 bis Beispiel 6

### Herstellung von erfindungsgemäßen O/W-Emulsionen nach dem PIT- Verfahren

Zur Herstellung der erfindungsgemäßen O/W-Emulsionen wurde die gesamte Menge an Wasser vorgelegt und auf 40 °C erhitzt. Unter moderatem Rühren wurden nacheinander Natriumbenzoat, Eumulgin® CO 40 bzw. 60 und Zitronensäure-Lösung zugegeben und gerührt, bis alles sich klar löste. In die klare Lösung wurden nacheinander Cremophor® WO 7 und die Ölkomponenten gemäß den Angaben der Tabelle 1 eingerührt. Die erhaltene O/W-Emulsionen wurden unter Rühren erhitzt bis zur vollständigen Phaseninversion Anschließend wurde mit einer Kühlrate von 1 bis 2 °C pro Minute auf Raumtemperatur (23 °C) abgekühlt. Bei 30 °C wurde der endgültige pH-Wert mit Zitronensäure-Lösung eingestellt auf 4,0 bzw. 3,5. Bei der Abkühlung erhielt man durch Phasenumkehr niedrigviskose, lagerstabile O/W-Emulsionen mit den in Tabelle 1 aufgeführten Eigenschaften.

Die Phaseninversionstemperatur ist der rechnerische Mittelwert des Temperaturbereiches beginnend mit der Temperatur, bei der die Leitfähigkeit sinkt bis zu der Temperatur, bei der die Leitfähigkeit gleich null ist. Die Leitfähigkeitsmessung erfolgt unter Verwendung einer 4-Elektroden-Leitfähigkeitsmesszelle Tetracon 325/S der Firma WTW.

Die Viskosität (in mPas) wurde ermittelt gemäß Brookfield/RVT/23°C+/-3°C/Sp 3/50rpm.

Der Öltröpfchendurchmesser in [µm] wurde bestimmt als Medianwert der Volumenverteilung mit dem Gerät: Beckman Coulter LS-230, Small Volume Modul;
Messinfos / Parameter: Mie-Streuung, mit PIDS Daten, Brechungsindex von Wasser: 1,332, Brechungsindex der Partikel: 1,47.

Der erfindungsgemäß bedeutsame Wert δ wurde berechnet als = Masse (Eumulgin® CO40 bzw. 60): (Masse (Eumulgin® CO40 bzw. 60)+Masse (Cremophor® WO7)).

Die erhaltenen Produkte wurden 4 Wochen bei -5 °C, 5 °C, Raumtemperatur (23 °C) und 40 °C gelagert. Die Formulierungen waren in Viskosität und Aussehen unverändert.

**Tabelle 1**

| **Beispielrezepturen** | | **Beispiel 1** | **Beispiel 2** | **Beispiel 3** | **Beispiel 4** | **Beispiel 5** | **Beispiel 6** |
|---|---|---|---|---|---|---|---|
| **Product** | **INCI** | **Gew.-%** | **Gew.-%** | **Gew.-%** | **Gew.-%** | **Gew.-%** | **Gew.-%** |
| Cetiol® AB | C12-15 Alkyl Benzoate | 10,0 | 20,0 | | | | |
| Luvitol® Lite | Hydrogenated Polyisobutene | 10,0 | | 20,0 | 20 | | |
| Cetiol® V | Decyloleate | | | | | 20 | |
| Myritol® 312 | Caprylic/Capric Triglyceride | | | | | | 20 |
| Eumulgin® CO 40 | PEG-40 Hydrogenated Castor Oil | 11,5 | 11,5 | 11,5 | 11,5 | 9,5 | 6,6 |
| Cremophor® WO 7 | PEG-7 Hydrogenated Castor Oil | 10,5 | 10,5 | 10,5 | 8,5 | 12,5 | 15,4 |
| Zitronensäure (50 Gew.-% Lösung) | Citric Acid | ca. 1,5 | ca. 1,5 | ca. 1,8 | ca. 1,5 | Ca. 1,5 | ca. 1,5 |
| Natriumbenzoat | Sodium Benzoate | 0,5 | 0,5 | 0,5 | 0,5 | 0,5 | 0,5 |
| Water demin. | | ad 100 | ad 100 | ad 100 | ad 100 | ad 100 | ad 100 |
| pH Wert | | 4,0 | 4,0 | 3,5 | 3,5 | 4,0 | 4,0 |
| PIT Phaseninversionstemperatur [°C] | | 67 | 71 | 69 | 76 | 69 | 71 |
| δ= | | 0,52 | 0,52 | 0,52 | 0,58 | 0,43 | 0,30 |
| Aussehen | | bläulich | bläulich | bläulich | bläulich | bläulich | bläulich |
| Teilchendurchmesser [µm] | | 0,094 | 0,076 | 0,089 | 0,089 | 0,087 | 0,080 |
| Viskosität [mPas]: | | 130 | 80 | 190 | 80 | 84 | 54 |
| Stabilität nach 2 Wochen Lagerung bei bei -5 °C RT 40°C | | **+** | **+** | **+** | **+** | **+** | **+** |
| | | **+** | **+** | **+** | **+** | **+** | **+** |
| | | **+** | **+** | **+** | **+** | **+** | **+** |

### Beispiele 7 bis 10 und Vergleichsbeispiele 1 und 2

Die Bedeutung des Emulgatorverhältnisses δ als Masse Eumulgin® CO40 bzw. 60: (Masse Eumulgin® CO40 bzw. 60 + Masse Cremophor® WO7) bei Cetiol® AB als Öl zeigt Tabelle 2. Es ist ersichtlich, dass bei δ kleiner 0,3 keine Phasenumkehr erfolgt und eine W/O-Emulsion vorliegt (Vergleichsbeispiel 1). Bei δ= 0,9 erfolgt keine Phasenumkehr bis 90 °C, da die PIT-Temperatur zu hoch liegt (Vergleichsbeispiel 2).

**Tabelle 2**

| **Rezepturen** | | **Beispiel 7** | **Beispiel 8** | **Beispiel 9** | **Beispiel 10** | **Vgl.bsp.1** | **Vgl.bsp.2** |
|---|---|---|---|---|---|---|---|
| **Product** | **INCl** | **Gew.-%** | **Gew.-%** | **Gew.-%** | **Gew.-%** | **Gew.-%** | **Gew.-%** |
| Cetiol® AB | C12-15 Alkyl Benzoate | 20,0 | 20,0 | 20 | 20 | 20 | 20 |
| Eumulgin® CO 40 | PEG-40 Hydrogenated Castor Oil | 9,5 | 14,0 | 16,0 | | 6,0 | 19,8 |
| Eumulgin® CO 60 | PEG-60 Hydrogenated Castor Oil | | | | 9,5 | | |
| Cremophor® WO 7 | PEG-7 Hydrogenated Castor Oil | 12,5 | 8,0 | 6,0 | 12,5 | 16,0 | 2,2 |
| Zitronensäure (50 Gew.-% Lösung) | Citric Acid | ca. 1,5 | ca. 1,5 | ca. 1,5 | ca. 1,5 | ca. 1,5 | ca. 1,5 |
| Natriumbenzoat | Sodium Benzoate | 0,5 | 0,5 | 0,5 | 0,5 | 0,5 | 0,5 |
| Water demin. | | ad 100 | ad 100 | ad 100 | ad 100 | ad 100 | ad 100 |
| pH Wert | | 4,0 | 4,0 | 4,0 | 4,0 | 4,0 | 4,0 |
| PIT Phaseninversionstemperatur [°C] | | 66 | 78 | 84 | 82 | Keine PIT, W/O-Emulsion | Keine Inversion bis 90 °C |
| δ= | | 0,43 | 0,64 | 0,73 | 0,43 | 0,27 | 0,90 |
| Aussehen | | bläulich | bläulich | bläulich | bläulich | weiß | weiß, aufgerahmt |
| Viskosität [mPas]: | | 80 | 110 | 120 | 102 | 330 | Nicht bestimmbar; Probe entmischt |
| Teilchendurchmesser [µm] | | 0,087 | 0,076 | 0,074 | 0,078 | > 2,0 | > 2,0 |

### Beispiel 11

### Anwendung der erfindungsgemäßen O/W-Emulsionen als sprühbare Wet Wipe Formulierung

Die erfindungsgemäße O/W-Emulsion nach Beispiel 2 wurde bei Raumtemperatur problemlos für die Anwendung verdünnt (Angaben in Gew.-%):

| | |
|---|---|
| O/W-Emulsion von Beispiel 2 | 2,0 |
| Wasser | ad 100 |
| pH | 5,5 |

Die Emulsionen blieben während der Verdünnung stabil; konnten bei Raumtemperatur mit Wasser verdünnt werden und sind sofort einsetzbar und sprühbar. Die Verdünnungen vergelen nicht und sind nach wie vor niedrigviskos (<200 mPas).

## Patentansprüche

1. Niedrigviskose O/W-Emulsionen mit Öl-Teilchendurchmesser von 0,01 bis 0,3 µm für kosmetische Anwendungen bestehend aus
(a) einem oder mehreren Öl(en)
(b) einer Emulgatormischung
(c) Wasser
(d) und ggf. kosmetischen Wirk- und/oder Hilfsstoffen,
**dadurch gekennzeichnet, dass** die Emulgatormischung besteht aus b1) Anlagerungsprodukten von 1 bis 15 Mol Ethylenoxid an hydriertem Ricinusöl und b2) Anlagerungsprodukten von 20 bis 80 Mol Ethylenoxid an hydriertem Ricinusöl in einem Masseverhältnis (= Emulgatorquotient) δ = Masse b2 : Masse (b1+ b2) im Bereich von 0,3 bis 0,8 und wobei die Öl(e) a) einen Polaritätsindex von 15 bis 55 [mN/m] aufweisen.

2. O/W-Emulsionen nach Anspruch 1, **dadurch gekennzeichnet, dass** b1) Anlagerungsprodukte von 3 bis 12 Mol, vorzugsweise 5 bis 10 Mol und insbesondere 7 Mol Ethylenoxid an hydriertem Ricinusöl sind.

3. O/W-Emulsionen nach Anspruch 1, **dadurch gekennzeichnet, dass** b2) Anlagerungsprodukte von 25 bis 60 Mol, vorzugsweise 35 bis 60 Mol und insbesondere 38 bis 45 Mol Ethylenoxid an hydriertem Ricinusöl sind.

4. O/W-Emulsionen nach Anspruch 1, **dadurch gekennzeichnet, dass** das oder die Öl(e) a) ausgewählt aus der von Triglyceriden von C6-C22 Fettsäuren, hydrierten Polyisobutenen, Benzoesäureester von Alkoholen mit 8 bis 22 Kohlenstoffatomen und Ester von C6-C22 Fettsäuren mit linearen C6-C22 Fettalkoholen gebildeten Gruppe.

5. O/W-Emulsionen nach einem der Ansprüche 1 bis 4, **dadurch gekennzeichnet, dass** der Öl-Teilchendurchmesser im Bereich von 0,06 bis 0,2 µm liegt.

6. O/W-Emulsionen nach einem der Ansprüche 1 bis 5, **dadurch gekennzeichnet, dass** sie Viskositäten im Bereich von 1 bis 300 mPas bestimmt gemäß Brookfield/RVT/ 23°C+/-3°C/Sp 3/50rpm aufweisen.

7. O/W-Emulsionen nach einem der Ansprüche 1 bis6, **dadurch gekennzeichnet, dass** die Öle
a) und die Emulgatormischung b) in einem Gewichtsverhältnis von 1:3 bis 3:1 vorliegen.

8. O/W-Emulsionen nach einem der Ansprüche 1 bis 7, **dadurch gekennzeichnet, dass** die Öle
a) Benzoesäureester von Alkoholen mit 8 bis 22 Kohlenstoffatomen sind und die Emulgatormischung b) aus b1) Anlagerungsprodukten von 7 Mol Ethylenoxid an hydriertem Ricinusöl und b2) Anlagerungsprodukten von 38 bis 45 Mol Ethylenoxid an hydriertem Ricinusöl besteht.

9. O/W-Emulsionen nach einem der Ansprüche 1 bis 8, **dadurch gekennzeichnet, dass** sie bestehen aus
a) 15 bis 22 Gew.-% Benzoesäureester von Alkoholen mit 8 bis 22 Kohlenstoffatomen
b) 18 bis 26 Gew.-% einer Emulgatormischung aus
b1) Anlagerungsprodukte von 7 Mol Ethylenoxid an hydriertem Ricinusöl und
b2) Anlagerungsprodukte von 38 bis 45 Mol Ethylenoxid an hydriertem Ricinusöl mit einem Emulgatorquotient δ im Bereich von 0,3 bis 0,8,
und 47 bis 76,5 Gew.-% Wasser
und 0,2 bis 5 Gew.-% kosmetische Wirk- und Hilfsstoffe.

10. O/W-Emulsionen nach einem der Ansprüche 1 bis 9, **dadurch gekennzeichnet, dass** die kosmetischen Wirk- und/oder Hilfsstoffe d) Konservierungsmittel und/oder pH-Regulantien sind.

11. Verfahren zur Herstellung von niedrigviskosen O/W-Emulsionen mit Öl-Teilchendurchmesser von 0,01 bis 0,3 µm nach Anspruch 1 nach dem Phaseninversionsverfahren, **dadurch gekennzeichnet, dass** man eine O/W-Emulsion mit Teilchendurchmesser über 2 µm bestehend aus
(a) einem oder mehreren Öl(en) mit einem Polaritätsindex von 15 bis 55 [mN/m]
(b) einer Emulgatormischung bestehend aus
b1) Anlagerungsprodukten von 1 bis 15 Mol Ethylenoxid an hydriertem Ricinusöl und
b2) Anlagerungsprodukten von 20 bis 80 Mol Ethylenoxid an hydriertem Ricinusöl in einem Masseverhältnis δ= Masse b2 : Masse (b1+b2) im Bereich von 0,3 bis 0,8
(c) Wasser
(d) und ggf. kosmetischen Wirk- und/oder Hilfsstoffen,
einsetzt, auf Temperaturen im Bereich von 40 °C bis 90 °C erhitzt und anschließend auf Raumtemperatur abgekühlt.

12. Verwendung der O/W- Emulsionen nach Anspruch 1 zur Imprägnierung von Textil und Papier für die Kosmetik, insbesondere von Wet Wipes.

13. Verwendung der O/W- Emulsionen nach Anspruch 12, **dadurch gekennzeichnet, dass** sie in Mengen von 0,2 bis 10 Gew.-% in Form einer wässrigen Verdünnung verwendet werden.

14. Wet Wipes für die Kosmetik imprägniert mit einer wässrigen Verdünnung einer O/W-Emulsion nach Anspruch 1.

## Claims

1. A low viscosity O/W emulsion with oil particle diameters of 0.01 to 0.3 µm for cosmetic applications consisting of
(a) one or more oil (s)
(b) an emulsifier mixture
(c) water
(d) and optionally cosmetic active compounds and/or additives,
**wherein** the emulsifier mixture consists of b1) addition products of 1 to 15 mol of ethylene oxide to hydrogenated castor oil and b2) addition products of 20 to 80 mol of ethylene oxide to hydrogenated castor oil in a mass ratio (= emulsifier quotient) δ= mass b2 : mass (b1+ b2) in the range from 0.3 to 0.8 and where the oil(s) a) have/has a polarity index of 15 to 55 [mN/m].

2. The O/W emulsion according to claim 1, **wherein** b1) are addition products of 3 to 12 mol, preferably 5 to 10 mol and in particular 7 mol of ethylene oxide to hydrogenated castor oil.

3. The O/W emulsion according to claim 1, **wherein** b2) are addition products of 25 to 60 mol, preferably 35 to 60 mol and in particular 38 to 45 mol of ethylene oxide to hydrogenated castor oil.

4. The O/W emulsion according to claim 1, **wherein** the oil (s) a) are selected from the group made up of triglycerides of C6-C22 fatty acids, hydrogenated polyisobutenes, benzoate esters of alcohols with 8 to 22 carbon atoms and esters of C6-C22 fatty acids with linear C6-C22 fatty alcohols.

5. The O/W emulsion according to one of claims 1 to 4, **wherein** the oil particle diameter lies in the range from 0.06 to 0.2 µm.

6. The O/W emulsion according to one of claims 1 to 5, **wherein** they have viscosities in the range from 1 to 300 mPas determined according to Brookfield/RVT/ 23°C+/-3°C/Sp 3/50 rpm.

7. The O/W emulsion according to one of claims 1 to 6, **wherein** the oils a) and the emulsifier mixture b) are present in a weight ratio of 1:3 to 3:1.

8. The O/W emulsions according to one of claims 1 to 7, **wherein** the oils a) are benzoate esters of alcohols with 8 to 22 carbon atoms and the emulsifier mixture b) consists of b1) addition products of 7 mol of ethylene oxide to hydrogenated castor oil and b2) addition products of 38 to 45 mol of ethylene oxide to hydrogenated castor oil.

9. The O/W emulsion according to one of claims 1 to 8, **wherein it** consists of
a) 15 to 22 wt % of benzoate esters of alcohols with 8 to 22 carbon atoms
b) 18 to 26 wt % of an emulsifier mixture consisting of
b1) addition products of 7 mol of ethylene oxide to hydrogenated castor oil and
b2) addition products of 38 to 45 mol of ethylene oxide to hydrogenated castor oil with an emulsifier quotient in the range from 0.3 to 0.8. and 47 to 76.5 wt % of water
and 0.2 to 5 wt % of cosmetic active compounds and additives.

10. The O/W emulsion according to one of claims 1 to 9, **wherein** the cosmetic active compounds and/or additives d) are preservatives and/or pH regulators.

11. A process for producing low viscosity O/W emulsions with oil particle diameters of 0.01 to 0.3 µm according to claim 1 by the phase inversion process, **wherein** an O/W emulsion with particle diameters over 2 µm consisting of
(a) one or more oil (s) with a polarity index of 15 to 55 [mN/m]
(b) an emulsifier mixture consisting of
b1) addition products of 1 to 15 mol of ethylene oxide to hydrogenated castor oil and
b2) addition products of 20 to 80 mol of ethylene oxide to hydrogenated castor oil in a mass ratio δ= mass b2 : mass (b1+b2) in the range from 0.3 to 0.8
(c) water
(d) and optionally cosmetic active compounds and/or additives,
is used, heated to temperatures in the range from 40°C to 90°C and then cooled to room temperature.

12. The use of the O/W emulsion according to claim 1 for the impregnation of textile and paper for cosmetics, in particular of wet wipes.

13. The use of the O/W emulsion according to claim 12, **wherein** it is used in quantities of 0.2 to 10 wt.% in the form of an aqueous dilution.

14. A wet wipe for cosmetics impregnated with an aqueous dilution of an O/W emulsion according to claim 1.

## Revendications

1. Émulsions H/E de basse viscosité présentant un diamètre des particules d'huile de 0,01 à 0,3 µm pour des utilisations cosmétiques, constituées par
(a) une ou plusieurs huile(s),
(b) un mélange émulsifiant
(c) de l'eau et
(d) le cas échéant des substances actives et/ou des adjuvants cosmétiques,
**caractérisées en ce que** le mélange émulsifiant est constitué par b1) des produits d'addition de 1 à 15 moles d'oxyde d'éthylène sur de l'huile de ricin hydrogénée et b2) des produits d'addition de 20 à 80 moles d'oxyde d'éthylène sur de l'huile de ricin hydrogénée dans un rapport massique (= quotient d'émulsifiants) δ = masse b2:masse (b1+b2) dans la plage de 0,3 à 0,8 et la/les huile(s) présentant un indice de polarité de 15 à 55 [mN/m].

2. Émulsions H/E selon la revendication 1, **caractérisées en ce que** b1) sont des produits d'addition de 3 à 12 moles, de préférence de 5 à 10 moles et en particulier de 7 moles d'oxyde d'éthylène sur de l'huile de ricin hydrogénée.

3. Émulsions H/E selon la revendication 1, **caractérisées en ce que** b2) sont des produits d'addition de 25 à 60 moles, de préférence de 35 à 60 moles et en particulier de 38 à 45 moles d'oxyde d'éthylène sur de l'huile de ricin hydrogénée.

4. Émulsions H/E selon la revendication 1, **caractérisées en ce que** la ou les huile (s) a) est/sont choisie(s) dans le groupe formé par les triglycérides d'acides gras en C6-C22, les polyisobutènes hydrogénés, les esters d'acide benzoïque d'alcools comprenant 8 à 22 atomes de carbone et les esters d'acides gras en C6-C22 avec des alcools gras linéaires en C6-C22.

5. Émulsions H/E selon l'une quelconque des revendications 1 à 4, **caractérisées en ce que** le diamètre des particules d'huile se situe dans la plage de 0,06 à 0,2 µm.

6. Émulsions H/E selon l'une quelconque des revendications 1 à 5, **caractérisées en ce qu'**elles présentent des viscosités dans la plage de 1 à 300 mPa.s, déterminées selon Brookfield/RVT/ 23°C/-3°C/ module 3/50 t/min.

7. Émulsions H/E selon l'une quelconque des revendications 1 à 6, **caractérisées en ce que** les huiles a) et le mélange émulsifiant b) se trouvent dans un rapport pondéral de 1:3 à 3:1.

8. Émulsions H/E selon l'une quelconque des revendications 1 à 7, **caractérisées en ce que** les huiles a) sont des esters d'acide benzoïque d'alcools comprenant 8 à 22 atomes de carbone et le mélange émulsifiant b) est constitué par b1) des produits d'addition de 7 moles d'oxyde d'éthylène sur de l'huile de ricin hydrogénée et b2) des produits d'addition de 38 à 45 moles d'oxyde d'éthylène sur de l'huile de ricin hydrogénée.

9. Émulsions H/E selon l'une quelconque des revendications 1 à 8, **caractérisées en ce qu'**elles sont constituées par :
a) 15 à 22% en poids d'esters d'acide benzoïque d'alcools comprenant 8 à 22 atomes de carbone
b) 18 à 26% en poids d'un mélange émulsifiant constitué par
b1) des produits d'addition de 7 moles d'oxyde d'éthylène sur de l'huile de ricin hydrogénée et
b2) de produits d'addition de 38 à 45 moles d'oxyde d'éthylène sur de l'huile de ricin hydrogénée à un quotient d'émulsifiants δ dans la plage de 0,3 à 0,8,
et 47 à 76,5% en poids d'eau
0,2 à 5% en poids de substances actives et d'adjuvants cosmétiques.

10. Émulsions H/E selon l'une quelconque des revendications 1 à 9, **caractérisées en ce que** les substances actives et/ou les adjuvants cosmétiques d) sont des conservateurs et/ou des agents de régulation du pH.

11. Procédé pour la préparation d'émulsions H/E de basse viscosité, présentant un diamètre des particules d'huile de 0,01 à 0,3 µm selon la revendication 1 selon le procédé d'inversion de phases, **caractérisé en ce qu'**on utilise une émulsion H/E présentant un diamètre des particules supérieur à 2 µm, constituée par
(a) une ou plusieurs huile(s) présentant un indice de polarité de 15 à 55 [mN/m]
(b) un mélange émulsifiant constitué par
b1) des produits d'addition de 1 à 15 moles d'oxyde d'éthylène sur de l'huile de ricin hydrogénée et
b2) des produits d'addition de 20 à 80 moles d'oxyde d'éthylène sur de l'huile de ricin hydrogénée dans un rapport massique δ = masse b2:masse (b1+b2) dans la plage de 0,3 à 0,8,
(c) de l'eau
(d) et le cas échéant des substances actives et/ou des adjuvants cosmétiques,
on chauffe à des températures dans la plage de 40°C à 90°C puis on refroidit à température ambiante.

12. Utilisation des émulsions H/E selon la revendication 1 pour l'imprégnation de textile et de papier pour la cosmétique, en particulier de lingettes humides (Wet Wipes) .

13. Utilisation des émulsions H/E selon la revendication 12, **caractérisée en ce qu'**elles sont utilisées en des quantités de 0,2 à 10% en poids sous forme d'une dilution aqueuse.

14. Lingettes humides pour la cosmétique imprégnées par une dilution aqueuse d'une émulsion H/E selon la revendication 1.
